# FASCICULE DE BREVET EUROPEEN

(11) **EP 1 687 241 B1**
(45) Date de publication et mention de la délivrance du brevet: **02.05.2012**
(21) Numéro de dépôt: 04805429.0
(22) Date de dépôt: 09.11.2004
(51) Int. Cl.: B01J 29/70, B01J 29/74, C07C 5/27, C01B 39/48

(54) **UTILISATION D'UNE ZEOLITHE DE TYPE STRUCTURAL EUO CONTENANT LE STRUCTURANT HEXYLQUINUCLIDINIUM COMME CATALYSEUR**
VERWENDUNG VON ZEOLIT EUO MIT HEXYLCHINUCLIDINIUM-STRUKTURBILDNER ALS KATALYSATOR
USE OF EUO STRUCTURAL ZEOLITE CONTAINING AN HEXYLQUINUCLIDINIUM STRUCTURING AS CATALYST

(30) Priorité: 14.11.2003 FR 0313399
(43) Date de publication de la demande: 09.08.2006
(73) Titulaire: Institut Français du Pétrole, 92852 Rueil Malmaison Cedex (FR)
(72) Inventeur: ROULEAU, Loic, F-69390 Charly (FR); LACOMBE, Sylvie, F-69230 Saint Genis Laval (FR)
(86) Numéro de dépôt international: PCT/FR2004/002886
(87) Numéro de publication internationale: WO 2005/049494

(56) Documents cités:
- EP-A- 1 151 963
- EP-A- 1 151 964
- EP-A- 1 182 247
- GRUNEWALD-LUKE, A. ET AL: "Quinuclidine derivatives as structure directing agents for the synthesis of boron containing zeolites" JOURNAL OF MATERIALS CHEMISTRY , 9(10), 2529-2536 CODEN: JMACEP; ISSN: 0959-9428, 1999, XP002285909 cité dans la demande

## Description

### Domaine technique

La présente invention se rapporte à l'utilisation d'une zéolithe de type structural EUO contenant dans sa porosité intracristalline un cation organique azoté, plus précisément un cation alkylquinuclidinium, et ayant une composition chimique et une proportion d'azote spécifiques. L'invention se rapporte à l'utilisation de celle-ci comme solide acide dans un procédé de conversion de charges hydrocarbonées.

### Art antérieur

Les zéolithes de type structural EUO sont décrites dans l'art antérieur (W.M. Meier et D.H. Olson, "Atlas of Zeolites Structure types", 5ème Edition, 2001) et présente un réseau microporeux monodimensionnel, dont le diamètre des pores est de 4,1 x 5,7 Å (1 Å = 1 Angström = 1.10⁻¹⁰ m). N.A. Briscoe *et al* ont enseigné que ces canaux monodimensionnels possèdent des poches latérales de profondeur 8,1 Å et de diamètre 6,8 x 5,8 Å (Zeolites, 8, 74, 1988).

Les zéolithes de type structural EUO, répertoriées dans l'Atlas des Zéolithes, comprennent la zéolithe EU-1, la zéolithe TPZ-3 et la zéolithe ZSM-50 et ont généralement la formule suivante sous forme anhydre : 100 XO₂ ; 0-10 T₂O₃ ; 0-20 R_{2/n}O ; où R représente un cation de valence n, X représente le silicium et/ou le germanium, T représente au moins un élément choisi parmi l'aluminium, le fer, le gallium, le bore, le titane, le vanadium, le zirconium, le molybdène, l'arsenic, l'antimoine, le chrome et le manganèse.

De manière générale, les méthodes de préparation de zéolithes de type structural EUO comprennent le mélange en milieu aqueux d'au moins une source d'un élément X, d'au moins une source d'un élément T, d'une source d'un métal alcalin et d'un composé organique azoté jouant le rôle de structurant.

Les modes de synthèse des zéolithes de type structural EUO diffèrent en particulier par la nature du structurant organique, la proportion d'azote dans la zéolithe brute de synthèse et la composition chimique de la charpente cristalline.

La zéolithe EU-1, décrite par Casci dans la demande de brevet européenne EP-A-0 042 226, est préparée avec les sources X et T (X/T préférentiellement compris entre 5 et 75) en utilisant comme structurant soit le dérivé alkylé d'une polyméthylène α-m diammonium, soit un produit de dégradation dudit dérivé, soit encore des précurseurs dudit dérivé, le structurant se trouvant après synthèse dans la porosité intracristalline de ladite zéolithe (A. Moini et al, Zeolites, 14, 1994).

La zéolithe TPZ-3, décrite dans la demande de brevet européenne EP-A-0 051 318, est préparée avec les sources X et T (X/T compris entre 10 et 125) en utilisant la même famille de structurant que celle employée pour synthétiser la zéolithe EU-1. Il est notamment décrit l'utilisation du composé 1,6-N,N,N,N',N',N'-hexaméthylhexamémylènediammonium.

La zéolithe ZSM-50, décrite dans les documents EP-A-0 159 845 et US 4,640,829, est préparée avec les sources X et T (X/T supérieur à 50) en utilisant comme structurant le dérivé dibenzyldiméthylammonium (DBDMA), lequel est contenu dans la porosité intracristalline de ladite zéolithe (A. Thangaraj et al, Zeolites, 11, 1991).

La zéolithe de type structural EUO décrite dans la demande de brevet EP-A-1 151 963 présente un rapport X/T compris entre 5 et 50 et un rapport atomique N/T compris entre 0,010 et 0,065.

La zéolithe de type structural EUO décrite par A. Grünewald-Lüke et ai (J. Mater. Chem., 1999, 9, 2529-2536) est préparée avec le silicium et le bore en utilisant comme structurant le cation hexylquinuclidinium (HexylQ).

Les zéolithes sont très utilisées dans l'industrie du raffinage et de la pétrochimie, comme élément d'un catalyseur dans un procédé de conversion de charges hydrocarbonées, comme adsorbant pour le contrôle de la pollution et comme tamis moléculaire pour la séparation.

### Résumé de l'invention

La présente invention est basée sur l'utilisation d'une zéolithe de type structural EUO, comprenant au moins du silicium et au moins de l'aluminium, ladite zéolithe, sous sa forme brute de synthèse, étant caractérisée en ce qu'elle contient un cation alkylquinuclidinium (alkylQ) dans sa porosité intracristalline et en ce qu'elle présente un rapport atomique N/Si supérieur à 0,065, N représentant l'élément azote. Ladite zéolithe présente un rapport Si/Al compris entre 5 et 50, de préférence entre 6 et 40, de manière plus préférée entre 7 et 30. L'invention concerne l'utilisation de ladite zéolithe comme solide acide dans un procédé de conversion de charges hydrocarbonées.

### Intérêt de l'invention

La zéolithe de type structural EUO, contenant sous sa forme brute de synthèse, un cation alkylquinuclidinium dans sa porosité intracristalline, et utilisée ultérieurement comme solide acide dans un catalyseur, en association avec au moins un liant, au moins un métal choisi parmi les éléments du groupe VIII, ledit métal étant déposé de préférence sur le liant, présente des performances catalytiques améliorées en transformation d'hydrocarbures en termes d'activité et de sélectivité, comme par exemple en hydroisomérisation du n-heptane. En particulier, il a été découvert de manière surprenante qu'une zéolithe de type structural EUO contenant, sous sa forme brute de synthèse, un cation alkylquinuclidinium dans sa porosité intracristalline, conduit à un catalyseur plus sélectif et plus actif que ceux à base des zéolithes de type structural EUO connues dans l'état de la technique.

### Description de l'invention

La présente invention se rapporte à l'utilisation d'une zéolithe de type structural EUO, comprenant au moins du silicium et au moins de l'aluminium, ladite zéolithe, sous sa forme brute de synthèse, étant caractérisée en ce qu'elle contient un cation alkylquinuclidinium dans sa porosité intracristalline et en ce qu'elle présente un rapport atomique N/Si supérieur à 0,065, N représentant l'élément azote.

La structure de la zéolithe de type structural EUO utilisée selon l'invention est identifiée par diffractométrie des rayons X. Sa cristallinité est calculée à partir du diagramme de diffraction par comparaison avec une zéolithe de type structural EUO de référence. La cristallinité correspond au rapport de la surface des pics des solides analysés sur la surface des pics de la zéolithe de type structural EUO de référence, dans le domaine d'angle de diffraction 2θ = 8 à 40°. La zéolithe utilisée selon la présente invention présente un diagramme de diffraction conforme à celui des zéolithes de type structural EUO et elle présente une cristallinité supérieure à 80 %, de préférence supérieure à 85 % et de manière encore plus préférée supérieure à 90 %.

La composition chimique de la zéolithe de type structural EUO utilisée selon l'invention est déterminée par les techniques classiques d'analyse élémentaire. En particulier, les teneurs en silicium et en aluminium, sont déterminées par fluorescence de rayons X. Le rapport Si/Al, de la zéolithe de type structural EUO utilisée selon l'invention est compris entre 5 et 50, de préférence entre 6 et 40 et de manière encore plus préférée entre 7 et 30. La teneur en azote est déterminée par détection catharométrique après une combustion et une réduction. Le rapport atomique N/Si, de la zéolithe de type structural EUO, sous sa forme brute de synthèse, utilisée selon l'invention comme solide acide après calcination est strictement supérieur à 0,065, N représentant l'élément azote.

Le cation alkylquinuclidinium jouant le rôle de structurant et présent dans la porosité intracristalline de la forme brute de synthèse de la zéolithe de type structural EUO, utilisée selon l'invention comme solide acide après calcination est identifié par spectroscopie de résonance magnétique nucléaire du carbone 13 à l'angle magique sous polarisation croisée. Le structurant organique azoté est le cation hexylquinuclidinium. Le spectre RMN ¹³C du composé organique présent dans la zéolithe de type structural EUO correspond à celui du dérivé hexylquinuclidinium.

Le cation alkylquinuclidinium contenu dans la porosité intracristalline de la forme brute de synthèse de la zéolithe utilisée selon l'invention comme solide acide après calcination est un cation organique azoté, noté dans la suite de l'exposé de l'invention par alkylQ, et a pour formule C₇H₁₃N-R⁺, où le radical R comporte six atomes de carbone : l'hexylquinuclidinium (hexylQ) de formule C₇H₁₃N-C₆H₁₃⁺.

Le procédé de préparation de la zéolithe de type structural EUO utilisée selon l'invention comprend le mélange en milieu aqueux d'au moins une source de silicium, d'au moins une source d'aluminium, le structurant organique azoté Q hexylquinuclidinium (hexylQ) de formule C₇H₁₃N-C₆H₁₃⁺.

Le structurant organique peut être par exemple un sel d'alkylquinuclidinium, en particulier un halogénure, un hydroxyde, un sulfate, un silicate ou un aluminate d'alkylquinuclidinium.

Les sels d'alkylquinuclidinium peuvent également être obtenus à partir de précurseurs. Des précurseurs particulièrement appropriés sont la quinuclidine et un halogénure d'alkyle ou un alcool primaire. S'agissant de l'hexylquinuclidinium, on utilisera avantageusement comme précurseurs la quinuclidine et l'halogénure d'hexyle ou l'hexanol. Ils peuvent être utilisés tels quels dans le mélange réactionnel ou peuvent être préchauffés ensemble dans le récipient de réaction, de préférence en solution avant l'addition des autres réactifs nécessaires pour la synthèse de la zéolithe de type structural EUO.

Des matériaux zéolithiques S jouant le rôle de germes peuvent être introduits lors du procédé de préparation sous plusieurs formes pour favoriser et accélérer la formation de la zéolithe de type structural EUO. Ces germes sont au moins en partie, et préférentiellement en totalité, de même type structural que la zéolithe EUO utilisée selon l'invention. Très avantageusement, il s'agit donc de germes d'au moins un matériau zéolithique de type structural EUO. Ces germes comprennent au moins une source d'élément X et au moins une source d'élément T, avec un rapport X/T compris entre 1 et 1000, où X est choisi parmi le du silicium et le germanium et T est choisi parmi de l'aluminium, le fer, le gallium, le titane, le vanadium, le zirconium, le molybdène, l'arsenic, l'antimoine, le chrome et le manganèse. Avantageusement, les germes de zéolithe présentent une composition chimique proche de celle de la zéolithe de type structural EUO utilisée selon l'invention à synthétiser, c'est-à-dire que lesdits germes présentent un rapport X/T, de préférence Sl/Al, proche de celui de la zéolithe de type structural EUO à synthétiser. De façon très préférée, la zéolithe utilisée selon l'invention est synthétisée à l'aide de germes zéolithiques comprenant les mêmes éléments X et T. Ces germes peuvent être introduits, après avoir subi au moins l'une des étapes choisie parmi les étapes suivantes : lavage, séchage, calcination et échange ionique. Les germes peuvent également être introduits sous la forme brute de synthèse.

Les matériaux zéolithiques jouant le rôle de germes peuvent être introduits à n'importe quel moment du procédé de préparation de la zéolithes que l'on cherche à synthétiser. Les germes peuvent être introduits en même temps que les sources de silicium et d'aluminium, que le structurant organique Q, ou les germes peuvent être introduits en premier dans le mélange aqueux ou encore les germes peuvent être introduits après l'introduction des sources de silicium et d'aluminium et du structurant. De préférence, les germes sont introduits après homogénéisation au moins en partie du mélange aqueux contenant les sources de silicium et d'aluminium et ledit structurant organique.

La taille des particules de germes zéolithiques pouvant avoir une influence sur le processus de synthèse, il convient de choisir des germes présentant une taille de particules telles que les conditions de synthèse soient optimales. On entend par particule de germes zéolithiques, soit un cristal de zéolithe soit un agrégat de cristaux de zéolithe où un agrégat est un ensemble formé par au moins deux cristaux de zéolithe ayant au moins un point de contact entre eux. Ainsi, au moins la majeure partie, c'est-à-dire au moins 90 % en volume, des particules de germes introduites lors de la préparation de la zéolithe de type structural EUO ont une taille comprise entre 0,001 et 500 µm, de préférence entre 0,005 et 250 µm et de manière encore plus préférée entre 0,005 et 200 µm.

Dans une mise en oeuvre particulière, indépendante ou non de la mise en oeuvre précédente, il est avantageux d'ajouter au milieu réactionnel au moins un sel de métal alcalin ou d'ammonium P. On peut citer par exemple des radicaux acides forts tels que du bromure, du chlorure, de l'iodure, du sulfate, du phosphate ou du nitrate, ou des radicaux acides faibles tels que les radicaux acides organiques, par exemple du citrate ou de l'acétate. Ce sel peut accélérer la cristallisation de la zéolithe de type structural EUO à partir du mélange réactionnel.

Dans le procédé de préparation, le mélange réactionnel a la composition suivante, exprimée sous la forme d'oxyde :

| | |
|---|---|
| SiO₂/Al₂O₃(mol/mol) | 10-100 |
| OH⁻/SiO₂ (mol/mol) | 0,002 à 2,0 |
| Q/SiO₂ (mol/mol) | 0,002 à 2,0 |
| Q/(M⁺ + Q) (mol/mol) | 0,1 à 1,0 |
| H₂O/SiO₂ (mol/mol) | 1 à 500 |
| P/SiO₂ (mol/mol) | 0 à 5 |
| S/SiO₂ (g/g) | 0 à 0,1 |

de manière préférée, le mélange réactionnel a la composition suivante, exprimée sous la forme d'oxydes :

| | |
|---|---|
| SiO₂/Al₂O₃(mol/mol) | 12-80 |
| OH⁻/SiO₂ (mol/mol) | 0,005 à 1,5 |
| Q/SiO₂ (mol/mol) | 0,005 à 1,5 |
| Q/(M⁺ + Q) (mol/mol) | 0,2 à 1,0 |
| H₂O/SiO₂ (mol/mol) | 3 à 250 |
| P/SiO₂ (mol/mol) | 0 à 1 |
| S/SiO₂ (g/g) | 0,0001 à 0,07 |

et, de manière encore plus préférée, le mélange réactionnel a la composition suivante, exprimée sous la forme d'oxydes :

| | |
|---|---|
| SiO₂/Al₂O₃ (mol/mol) | 14-60 |
| OH⁻/SiO₂ (mol/mol) | 0,01 à 1 |
| Q/SiO₂ (mol/mol) | 0,01 à 1 |
| Q/(M⁺ + Q) (mol/mol) | 0.3 à 1.0 |
| H₂O/SiO₂ (mol/mol) | 5 à 100 |
| P/SiO₂ (mol/mol) | 0 à 0,25 |
| S/SiO₂ (g/g) | 0,0001 à 0,04 |

où
M⁺ représente un métal alcalin,
Q représente le structurant organique ou les précurseurs dudit structurant,
S représente les germes de zéolithe présents sous la forme brute, séchée, calcinée ou échangée,
P représente le sel de métal alcalin ou d'ammonium.

M et/ou Q peuvent être présents sous forme d'hydroxydes ou de sels d'acides inorganiques ou organiques à la condition que le critère OH⁻ SiO₂ soit satisfait.

Le métal alcalin (M⁺) préféré est le sodium.

La source de silicium peut être tout composé comprenant le silicium et pouvant libérer cet élément en solution aqueuse sous forme réactive. La source de silicium peut être l'une quelconque de celles dont l'utilisation est habituellement envisagée pour la synthèse des zéolithes, par exemple la silice solide en poudre, l'acide silicique, la silice colloïdale ou la silice en solution. Parmi les silices en poudre utilisables, il convient de citer les silices précipitées, spécialement celles obtenues par précipitation à partir d'une solution d'un silicate de métal alcalin, comme les " Zeosil " ou les " Tixosil ", produites par Rhodia, les silices pyrogénées telles que les "Aerosil " produites par Degussa et les "Cabosil" produites par Cabot et les gels de silice. Des silices colloïdales de diverses granulométries peuvent être utilisées, comme celles vendues sous les marques déposées "LUDOX" de Dupont, et "SYTON" de Monsanto. Les silices dissoutes utilisables sont notamment les verres solubles ou silicates commercialisés contenant : 0,5 à 6,0 et spécialement 2,0 à 4,0 moles de SiO₂ par mole d'oxyde de métal alcalin et les silicates obtenues par dissolution de silice dans un hydroxyde de métal alcalin, un hydroxyde d'ammonium quaternaire ou un mélange de ceux-ci.

La source de l'aluminium peut être tout composé comprenant l'aluminium et pouvant libérer cet élément en solution aqueuse sous forme réactive. La source d'aluminium est le plus avantageusement l'aluminate de sodium, mais peut aussi être l'aluminium, un sel d'aluminium, par exemple le chlorure, le nitrate ou le sulfate, un alcoolate d'aluminium ou l'alumine elle-même qui de préférence se trouve sous une forme hydratée ou hydratable comme l'alumine colloïdale, la pseudoboehmite, la boehmite, l'alumine gamma, ou les trihydrates.

On peut utiliser des mélanges des sources citées ci-dessus. Des sources combinées de silicium et d'aluminium peuvent aussi être mises en oeuvre telles que les silice-alumines amorphes ou certaines argiles.

Le mélange de réaction est habituellement mis à réagir sous la pression autogène, éventuellement avec apport d'un gaz, par exemple d'azote, à une température comprise entre 85 et 250°C jusqu'à ce qu'il se forme des cristaux de la zéolithe, ce qui peut durer de 1 minute à plusieurs mois, de préférence de 2 à 30 jours, suivant la composition des réactifs, le mode de chauffage et de mélange, la température de travail et l'agitation. L'agitation est facultative, mais préférable, en particulier parce qu'elle abrège la durée de réaction.

Au terme de la réaction, la phase solide est collectée sur un filtre et lavée. A ce stade là, la zéolithe de type structural EUO obtenue selon le procédé est dite brute de synthèse et contient dans sa porosité intracristalline le cation hexylquinuclidinium. La zéolithe est alors prête pour les opérations suivantes comme le séchage, la calcination et l'échange d'ions. Après la réalisation d'une étape finale de calcination, la zéolithe de l'invention, est débarrassée du structurant organique azoté mais son rapport Si/Al reste Inchangé. Il va de soi que, sous forme calcinée, la zéolithe utilisée selon invention ne contient plus d'azote.

Ainsi, afin d'obtenir la forme hydrogène de la zéolithe de type structural EUO, on peut effectuer un échange d'ions avec un acide, spécialement un acide minéral fort comme l'acide chlorhydrique, sulfurique ou nitrique, ou avec un composé tel que le chlorure, le sulfate ou le nitrate d'ammonium. L'échange d'ions peut être effectué par dilution en une ou plusieurs fois avec la solution d'échange d'ions. La zéolithe peut être calcinée avant ou après l'échange d'ions, ou entre deux étapes d'échange d'ions, de préférence avant l'échange d'ions afin d'éliminer toute substance organique incluse, dans la mesure où l'échange d'ions s'en trouve facilité.

En règle générale, le ou les cations de la zéolithe de type structural EUO, peuvent être remplacés par un ou des cations quelconques de métaux et en particulier ceux des groupes IA, IB, IIA, IIB, IIIA, IIIB (y compris les terres rares), VIII (y compris les métaux nobles) de même que par le plomb, l'étain et le bismuth (tableau périodique dans "Handbook of Physic and Chemistry", 76ème édition). L'échange est réalisé au moyen de sels hydrosolubles quelconques contenant le cation approprié.

La présente invention concerne l'utilisation de la zéolithe de type structural EUO comme solide acide pour la catalyse dans les domaines du raffinage et de la pétrochimie, par solide acide, on entend que la zéolithe est sous forme hydrogène, c'est-à-dire que la zéolithe brute de synthèse a été calcinée et échangée.

Par exemple, lorsque la zéolithe de type structural EUO est utilisée comme solide acide dans un catalyseur, elle est sous forme calcinée, c'est-à-dire débarrassée du structurant azoté, et peut être associée à une matrice inorganique qui peut être inerte ou catalytiquement active et à une phase métallique. La matrice inorganique peut être présente simplement comme liant pour maintenir ensemble les petites particules de la zéolithe sous les différentes formes connues des catalyseurs (extrudés, pastilles, billes, poudres), ou bien peut être ajoutée comme diluant pour imposer le degré de conversion dans un procédé qui progresserait sinon à une allure trop rapide conduisant à un encrassement du catalyseur en conséquence d'une formation importante de coke. Des matrices inorganiques typiques sont notamment des matières de support pour les catalyseurs comme les différentes formes de silice, d'alumine, les silices-alumine, la magnésie, la zircone, les oxydes de titane, de bore, les phosphates d'aluminium, de titane, de zirconium, les argiles telles que le kaolin, la bentonite, la montmorillonite, la sépiolite, l'attapulgite, la terre à foulon, les matières poreuses synthétiques comme SiO₂-Al₂O₃, SiO₂-Zro₂, SiO₂-ThO₂, SiO₂-BeO, SiO₂-TiO₂ ou toute combinaison de ces composés.

La zéolithe de type structural EUO de l'invention peut aussi être associée à au moins une autre zéolithe et jouer le rôle de phase active principale ou d'additif.

La matrice inorganique peut être un mélange de différents composés, en particulier d'une phase inerte et d'une phase active.

La phase métallique est introduite sur la zéolithe seule, la matrice inorganique seule ou l'ensemble matrice inorganique-zèolithe par échange d'ions ou imprégnation avec des cations ou oxydes choisis parmi les éléments suivants : Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Ru, Rh, Os, Ir et tout autre élément de la classification périodique des éléments.

Les compositions catalytiques comportant la zéolithe de type structural EUO utilisée comme solide acide selon l'invention conviennent de façon générale à la mise en oeuvre des principaux procédés de transformation d'hydrocarbures et des réactions de synthèse de composés organiques tels que les éthers.

Les compositions catalytiques comportant la zéolithe de type structural EUO utilisée comme solide acide selon l'invention, trouvent avantageusement leur application dans les réactions d'isomérisation, de transalkylation et de dismutation, d'alkylation et de désalkylation, d'hydratation et de déshydratation, d'oligomérisation et de polymérisation, de cyclisation, d'aromatisation, de craquage et d'hydrocraquage, de reformage, d'hydrogénation et de déshydrogénation, d'oxydation, d'halogénation, de synthèses d'amines, d'hydrodésulfuration et d'hydrodénitrification, d'élimination catalytique des oxydes d'azote, lesdites réactions comprenant des hydrocarbures aliphatiques saturés et insaturés, des hydrocarbures aromatiques, des composés organiques oxygénés et des composés organiques contenant de l'azote et/ou du soufre, ainsi que des composés organiques contenant d'autres groupes fonctionnels.

Lorsque ladite zéolithe EUO est utilisée comme solide acide dans des réactions catalytiques, le catalyseur contient :
- au moins une zéolithe de type structural EUO telle que décrite ci-dessus, sous sa forme calcinée, et présentant un rapport Si/Al compris entre 5 et 50, de préférence entre 6 et 40 et de manière encore plus préférée entre 7 et 30,
- au moins un métal du groupe VIII, choisi de préférence dans le groupe constitué par le palladium et le platine et de manière encore plus préférée le platine,
- au moins un liant, de préférence l'alumine,
- éventuellement au moins un élément appartenant au groupe formé par les éléments des groupes IB, IIB, IIIA, IVA, VIB et VIIB, choisi dans le groupe formé par l'étain et l'indium,
- éventuellement du soufre.

De manière plus précise, le catalyseur comprend généralement par rapport au poids de catalyseur :
- de 1 à 90 % bornes incluses, de préférence de 3 à 75 % bornes incluses et de manière encore plus préférée de 4 à 60 % bornes incluses en poids, d'au moins une zéolithe de type structural EUO telle que décrite ci-dessus, sous sa forme calcinée, comprenant au moins du silicium et au moins de l'aluminium, dont le rapport atomique Si/Al est compris entre 5 et 50, de préférence entre 6 et 40 et de manière plus préférée entre 7 et 30, ladite zéolithe se présentant au moins en partie sous forme acide c'est-à-dire sous forme hydrogène H,
- de 0,01 à 10% bornes incluses, de préférence de 0,01 à 2 % bornes incluses et de manière encore plus préférée de 0,05 à 1,0 % bornes incluses en poids, d'au moins un métal du groupe VIII de la classification périodique des éléments, de préférence choisi dans le groupe formé par le platine et le palladium et de manière encore plus préférée le platine,
- éventuellement de 0,01 à 10 % bornes incluses, de préférence de 0,01 à 2% bornes incluses et de manière encore plus préférée entre 0,05 et 1,0 % bornes incluses en poids, d'au moins un élément du groupe formé par les groupes IB, IIB, IIIA, IVA, VIB et VIIB de la classification périodique des éléments, de préférence choisi dans le groupe formé par l'étain et l'indium,
- éventuellement du soufre dont la teneur est telle que le rapport du nombre d'atomes de soufre sur le nombre d'atomes de métal du groupe VIII déposés est compris entre 0,5 et 2 bornes incluses,
- le complément à 100 % en poids d'au moins un liant, de préférence de l'alumine.

Toute méthode de mise en forme convient pour le présent catalyseur. On pourra utiliser, par exemple, le pastillage ou l'extrusion ou la mise sous forme de billes. La mise en forme du catalyseur est généralement telle que le catalyseur est de préférence sous forme d'extrudés ou de billes en vue de son utilisation.

La zéolithe de type structural EUO utilisée selon l'invention, est traitée par au moins une étape de calcination, puis soumise à au moins un échange ionique dans au moins une solution de NH₄NO₃ de manière à obtenir une zéolithe dont la teneur en élément alcalin résiduel, par exemple le sodium, est plus ou moins importante.

La zéolithe de type structural EUO utilisée selon l'invention, sous sa forme calcinée, et comprise dans des compositions catalytiques, est au moins en partie, de préférence pratiquement totalement, sous forme acide, c'est-à-dire sous forme hydrogène (H⁺), la teneur en élément alcalin, par exemple le sodium, étant de préférence telle que le rapport atomique M/Al est inférieur à 0,5, de préférence inférieur à 0,1, de manière encore plus préférée inférieur à 0,02.

Le liant (ou matrice) compris dans le catalyseur à base de la zéolithe de type structural EUO consiste généralement en au moins un élément choisi dans le groupe formé par les argiles, la magnésie, les alumines, les silices, l'oxyde de titane, l'oxyde de bore, la zircone, les phosphates d'aluminium, les phosphates de titane, les phosphates de zirconium et les silices alumines. De préférence le liant est une alumine.

Les métaux peuvent être introduits soit tous de la même façon soit par des techniques différentes, à tout moment de la préparation, avant ou après mise en forme et dans n'importe quel ordre. De plus, des traitements intermédiaires tels que par exemple une calcination et/ou une réduction peuvent être appliqués entre les dépôts des différents métaux.

La préparation du catalyseur pourra être effectuée par toute méthode connue de l'homme du métier. Au moins un élément du groupe VIII est introduit dans la zéolithe ou sur le liant, de préférence sur le liant avant ou après mise en forme.

Une méthode préférée consiste à réaliser un mélange de la matrice et de la zéolithe suivie d'une mise en forme. La mise en forme est généralement suivie d'une calcination, généralement à une température comprise entre 250°C et 600°C bornes incluses. Au moins un élément du groupe VIII de la classification périodique des éléments est introduit après cette calcination, de préférence par dépôt sélectif sur le liant. Lesdits éléments sont déposés pratiquement à plus de 90 % bornes incluses totalement sur le liant de la manière connue de l'homme du métier par contrôle des paramètres utilisés lors dudit dépôt, comme par exemple la nature du précurseur utilisé pour effectuer ledit dépôt.

Au moins un élément du groupe VIII est déposé de manière préférée sur le mélange zéolithe EUO-liant préalablement mis en forme par tout procédé connu de l'homme du métier. Un tel dépôt est par exemple effectué par la technique d'imprégnation à sec, d'imprégnation par excès ou d'échange ionique. Tous les précurseurs conviennent pour le dépôt de ces éléments. De préférence, on mettra en oeuvre un échange anionique avec de l'acide hexachloroplatinique et/ou de l'acide hexachloropalladique en présence d'un agent compétiteur, par exemple l'acide chlorhydrique. Dans ce cas, le métal est pratiquement à plus de 90 % déposé totalement sur le liant et il présente une bonne dispersion et une bonne répartition macroscopique à travers le grain de catalyseur ce qui constitue une méthode préférée de préparation.

Une autre méthode préférée de préparation du catalyseur, utilisée dans la présente invention consiste à malaxer la zéolithe EUO, sous sa forme calcinée, dans un gel humide de matrice (obtenu généralement par mélange d'au moins un acide et d'une poudre de matrice), par exemple l'alumine, pendant une durée nécessaire à l'obtention d'une bonne homogénéité de la pâte ainsi obtenue, soit par exemple pendant une dizaine de minutes, puis à passer la pâte à travers une filière pour former des extrudés. Puis après séchage, par exemple pendant quelques heures à environ 120°C en étuve et après calcination, par exemple pendant deux heures à environ 500°C, au moins un élément, par exemple le platine, est déposé, par exemple par échange anionique avec de l'acide hexachloroplatinique en présence d'un agent compétiteur (par exemple l'acide chlorhydrique), ledit dépôt étant suivi d'une calcination par exemple pendant environ 2 heures à environ 500°C.

Eventuellement, au moins un autre élément choisi dans le groupe formé par les éléments des groupes IB, IIS, IIIA, IVA, VIB et VIIB est ajouté. On peut ajouter les éléments du groupe VIII et des groupes IB, IIB, IIIA, IVA, VIB et VIIB soit séparément à n'importe quelle étape de la préparation dudit catalyseur soit simultanément dans au moins une étape unitaire. Lorsqu'au moins un élément des groupes IB, IIB, IIIA, IVA, VIB et VIIB est ajouté séparément, il est avantageux de l'ajouter préalablement à l'élément du groupe VIII. Toutes les techniques de dépôt connues de l'homme du métier et tous les précurseurs conviennent.

Le platine est généralement introduit dans la matrice sous forme d'acide hexachloroplatinique, mais pour tout métal noble peuvent être également utilisés des composés ammoniaqués avec, dans ce cas, dépôt du métal noble dans la zéolithe, ou peuvent être utilisés des composés tels que, par exemple, le chloroplatinate d'ammonium, le dichlorure de platine dicarbonyle, l'acide hexahydroxyplatinique, le chlorure de palladium ou le nitrate de palladium.

Dans le cas du platine, on peut également citer par exemple les sels de platine Il tétramines de formule Pt(NH₃)₄X₂ ; les sels de platine IV hexamines de formule Pt(NH₃)₆X₄ ; les sels de platine IV halogénopentamines de formule (PtX(NH₃)₅)X₃ ; les sels de platine IV tétrahalogénodiamines de formule PtX₄(NH₃)₂ ; les complexes de platine avec les halogènes-polycétones et les composés halogénés de formule H(Pt(acac)₂X) ; X étant un halogène choisi dans le groupe formé par le chlore, le fluor, le brome et l'iode, et de préférence X étant le chlore, et acac représentant le groupe C₅H₇O₂ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques contenant de 4 à 12 atomes de carbone, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

L'élément additionnel, éventuellement introduit en plus, choisi dans le groupe formé par les éléments des groupes IB, IIS, IIIA, IVA, VIS et VIIB, peut être introduit par l'intermédiaire de composés tels que par exemple les chlorures, les bromures et les nitrates, les alkyles d'éléments des groupes IS, IIB, IIIA, IVA, VIS et VIIB, soit par exemple l'étain et l'indium, les alkyl étain, le nitrate et le chlorure d'indium.

Cet élément peut également être introduit sous la forme d'au moins un composé organique choisi dans le groupe constitué par les complexes dudit élément, en particulier les complexes polycétoniques du métal et les hydrocarbylmétaux tels que les alkyles, les cycloalkyles, les aryles, les alkylaryles métaux. Dans ce dernier cas, l'introduction du métal est avantageusement effectuée à l'aide d'une solution du composé organométallique dudit métal dans un solvant organique. On peut également employer des composés organohalogénés du métal. Comme composés du métal, on peut citer en particulier le tétrabutylétain dans le cas de l'étain, et le triphénylindium dans le cas de l'indium, Le solvant d'imprégnation est choisi dans le groupe constitué par les hydrocarbures paraffiniques, naphténiques ou aromatiques contenant de 4 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

Le métal additionnel peut être éventuellement introduit à tout moment de la préparation, de préférence préalablement au dépôt d'un ou plusieurs métaux du groupe VIII. Si ce métal est introduit avant le métal noble, le composé du métal utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal noble, on procède à une calcination sous air.

La préparation du catalyseur comprend généralement une calcination, habituellement à une température comprise entre environ 250°C et 600°C bornes incluses, pour une durée d'environ 0,5 à 10 heures, de préférence précédée d'un séchage, par exemple à l'étuve, à une température comprise entre la température ambiante et 250°C, de préférence entre 40 et 200°C. Ladite étape de séchage est de préférence menée pendant la montée en température nécessaire pour effectuer ladite calcination.

Dans le cas où le catalyseur de la présente invention contient du soufre, le soufre est introduit sur le catalyseur mis en forme, calciné, contenant le ou les métaux cités précédemment, soit *in-situ* avant la réaction catalytique, soit *ex-situ.* La sulfuration éventuelle intervient après la réduction. Dans le cas d'une sulfuration in situ. la réduction, si le catalyseur n'a pas été préalablement réduit, intervient avant la sulfuration. Dans le cas d'une sulfuration ex-situ, on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

La zéolithe de type structural EUO telle que décrite ci-dessus est très avantageusement utilisée comme solide acide dans une réaction d'hydroisomérisation du n-heptane après calcination de ladite zéolithe. Ladite réaction mise en oeuvre, en présence d'hydrogène, est généralement réalisée selon les conditions opératoires suivantes :
- une température comprise entre 150°C et 500°C bornes incluses,
- un rapport molaire du nombre de moles d'hydrogène sur le nombre de moles d'hydrocarbures ente 0.1 et 3.
- une pression totale comprise entre 0,1 et 4 MPa bornes incluses,
- une vitesse spatiale d'alimentation, exprimée en kilogramme de charge introduite par kilogramme de catalyseur et par heure, comprise entre 0,5 et 2 h⁻¹ bornes incluses.

L'invention est illustrée par les exemples suivants.

### EXEMPLE 1 (comparatif) : Zéolithe de type structural EUO contenant les éléments Si et Al, de rapport Si/Al égal à 25,0, synthétisée avec le cation hexaméthylammonium comme structurant organique.

La zéolithe de type structural EUO contenant les éléments Si et Al, de rapport Si/Al égal à 25,0 est synthétisée avec le bromure d'hexaméthonium (HM, 1,6 triméthylammonium-hexane) selon les conditions décrites par J.L. Casci *et al* dans l'exemple 3 du brevet EP-A- 0 042 226.

Les résultats de diffraction des rayons X et d'analyse chimique sont reportés dans le tableau 1. Le solide obtenu est une zéolithe EUO pure, de cristallinité de référence (100 %), de rapport Si/Al de 25,0.

**Tableau 1 : caractéristiques du solide obtenu**

| Diffraction des rayons X | |
|---|---|
| Identification de phase | EUO |
| Cristallinité (%) | 100 (référence) |

| Analyses chimiques | |
|---|---|
| SiO₂(% poids) | 81,0 |
| Al₂O₃ (% poids) | 2,75 |
| Na₂O (% poids) | 0,47 |
| N (% poids) | 1,40 |
| PAF (% poids) | 15,0 |
| Si/Al (mol/mol) | 25,0 |

| | |
|---|---|
| PAF = perte au feu | |

Cette zéolithe, contenant les éléments Si et Al, de rapport Si/Al de 25,0, préparée avec HM selon l'art antérieur, correspond à la zéolithe EU-1. Elle sert de germes pour la synthèse de la zéolithe de type structural EUO selon l'invention.

### EXEMPLE 2 (comparatif) : Zéolithe de type structural EUO contenant les éléments Si et Al, de rapport Si/Al égal à 26,0, synthétisée avec les précurseurs du cation dibenzyldiméthylammonium comme structurant organique.

La zéolithe de type structural EUO contenant les éléments Si et Al, de rapport Si/Al égal à 26,0 est synthétisée avec les précurseurs du chlorure de dibenzyldiméthylammonium (DBDMA), à savoir la benzyldiméthylamine (BDMA) et le chlorure de benzyle (BCI) selon les conditions décrites par L. Rouleau *et al* dans l'exemple 3 de la demande de brevet EP-A-1 151 963.

Les résultats de diffraction des rayons X et d'analyse chimique sont reportés dans le tableau 2. Le solide obtenu est une zéolithe de type structural EUO pure, de cristallinité de 100 % par rapport à la référence (zéolithe obtenue dans l'exemple 1), contenant les éléments Si et Al, de rapport Si/Al de 26,0 et contenant le cation DBDMA dans sa porosité intracristalline.

**Tableau 2 : caractéristiques du solide obtenu**

| Diffraction des rayons X | |
|---|---|
| Identification de phase | EUO |
| Cristallinité (%) | 100 |

| Analyses chimiques (teneurs en poids) | |
|---|---|
| SiO₂ (% poids) | 82,2 |
| Al₂O₃ (% poids) | 2,69 |
| Na₂O (% poids) | 0,54 |
| N (% poids) | 0,59 |
| PAF (% poids) | 14,3 |
| N/Si (mol/mol) | 0,031 |
| Si/Al (mol/mol) | 26,0 |

| Spectroscopie de résonance magnétique nucléaire du carbone 13 à l'angle magique sous polarisation croisée | |
|---|---|
| Identification du composé organique inclus dans la zéolithe | DBDMA |

| | |
|---|---|
| PAF = perte au feu | |

### EXEMPLE 3 (comparatif) : Zéolithe de type structural EUO contenant les éléments Si et B, de rapport Si/B égal à 11,7, synthétisée avec les précurseurs du cation hexylquinuclidinium comme structurant organique.

La zéolithe EUO contenant les éléments Si et B, de rapport Si/B égal à 11,7, est synthétisée avec les précurseurs du bromure d'hexylquinuclidinium (HexylQ), à savoir la quinuclidine (Q) et le bromure d'hexyle (HexylSr), selon les conditions décrites par Grünewald-Lüke et al dans la revue J. Mat. Chem, 1999, 9, 2529-2536.

Le tétraméthoxysilane (Sigma-Aldrich) est hydrolysé dans l'eau. A cette solution est ajouté l'hydroxyde d'hexylquinuclidinium, préparé par réaction de la quinuclidine (Sigma-Aldrich) avec l'iodure d'hexyle (Sigma-Aldrich) dans l'éthanol à température ambiante puis échange ionique avec un échangeur anionique, puis l'acide borique (Sigma-Aldrich). On fait réagir le mélange résultant dans un autoclave de 125 ml sous agitation pendant 63 jours à 180°C sous pression autogène. Après refroidissement, on filtre le produit et on le lave avec 100 ml d'eau déminéralisée puis on le sèche en étuve ventilée à 120°C. Les conditions de synthèse sont définies dans le tableau 3.

**Tableau 3 : conditions de synthèse.**

| Formulation du gel | |
|---|---|
| SiO₂ (mol) | 60 |
| B₂O₃ (mol) | 39 |
| HexylQOH (mol) | 60 |
| H₂O (mol) | 12600 |
| CH₃OH (mol) | 120 |

| Conditions de cristallisation | |
|---|---|
| Température (°C) | 180 |
| Durée (jours) | 63 |

| | |
|---|---|
| HexylQOH = hydroxyde d'hexylquinuclidinium | |

Les résultats de diffraction des rayons X et d'analyse chimique sont reportés dans le tableau 4. Le solide obtenu est une zéolithe de type structural EUO pure, de cristallinité de 95 % par rapport à la référence (zéolithe obtenue dans l'exemple 1), contenant les éléments Si et B, de rapport Si/B de 11,7.

**Tableau 4 : caractéristiques du solide obtenu**

| Diffraction des rayons X | |
|---|---|
| Identification de phase | EUO |
| Cristallinité (%) | 95 |

| Analyses chimiques | |
|---|---|
| SiO₂ (% poids) | 71,2 |
| B₂O₃ (% poids) | 3,6 |
| N (% poids) | 1,49 |
| PAF (% poids) | 25,2 |
| Si/B (mol/mol) | 11,7 |

| | |
|---|---|
| PAF = perte au feu | |

### EXEMPLE 4 (invention) : zéolithe de type structural EUO contenant les éléments Si et AI, de rapport Si/Al égal à 11,7, synthétisée avec les précurseurs du bromure d'hexylquinuclidinium comme structurant organique.

Les conditions de synthèse sont définies dans le tableau 5.

On prépare la solution A composée de silicium et des précurseurs de structurant en diluant 1,95 g de quinuclidine (Sigma-Aldrich) et 2,899 g de bromure d'hexyl (Sigma-Aldrich) dans 46,895 g d'eau puis en ajoutant 15,814 g de sol de silice colloïdale (Ludox HS40, Dupont, 40 % SiO₂). On dissout ensuite 0,204 g d'hydroxyde de sodium solide (Prolabo) et 0,974 g d'aluminate de sodium solide (Prolabo, 46 % Al₂O₃, 33 % Na₂O) dans 15,632 g d'eau pour former la solution B. On ajoute la solution B dans la solution A sous agitation puis 22,673 g d'eau. On mélange jusqu'à homogénéisation. On ajoute finalement 0,633 g de germes de zéolithe de type structural EUO, post synthèse, contenant les éléments Si, Al, le sodium et le cation hexaméthonium, préparée selon les conditions de l'art antérieur (solide ayant été obtenu dans l'exemple 1). On fait réagir le mélange résultant dans un autoclave de 125 ml sous agitation pendant 25 jours à 180°C sous pression autogène- Après refroidissement, on filtre le produit et on le lave avec 100 ml d'eau déminéralisée puis on le sèche en étuve ventilée à 120°C.

**Tableau 5 : conditions de synthèse**

| Formulation du gel | |
|---|---|
| SiO₂ (mol) | 60 |
| Al₂O₃ (mol) | 2,5 |
| Na₂O (mol) | 5 |
| Quinuclidine (mol) | 10 |
| HexylBr (mol) | 10 |
| H₂O (mol) | 3000 |
| EUO/SiO₂ (g/g) | 0,10 |

| Conditions de cristallisation | |
|---|---|
| Température (°C) | 180 |
| Durée (jour) | 25 |

| | |
|---|---|
| HexylBr = bromure d'hexyle | |

Les résultats de diffraction des rayons X, d'analyse chimique et de spectroscopie par résonance magnétique nucléaire du carbone 13 à l'angle magique sous polarisation croisée sont reportés dans le tableau 6. Cette synthèse conduit à la zéolithe de type structural EUO pure, de cristallinité de 95 % par rapport à la référence (zéolithe obtenue dans l'exemple 1), de rapport Si/Al de 11,7 et contenant le cation hexylquinuclidinium dans sa porosité intracristalline.

**Tableau 6 : caractéristiques du solide obtenu**

| Diffraction des rayons X | |
|---|---|
| Identification de phase | EUO |
| Cristallinité (%) | 95 |

| analyses chimiques | |
|---|---|
| SiO₂ (% poids) | 69,6 |
| Al₂O₃ (% poids) | 5,1 |
| Na₂O (% poids) | 0,59 |
| N (% poids) | 1,46 |
| PAF (% poids) | 24,7 |
| N/Si (mol/mol) | 0,089 |
| Si/Al (mol/mol) | 11,7 |

| Spectroscopie de résonance magnétique nucléaire du carbone 13 à l'angle magique sous polarisation croisée (déplacements chimiques) | |
|---|---|
| Identification du composé organique inclus dans la zéolithe | hexylquinuclidinium |

Cette zéolithe brute de synthèse de type structural EUO, contenant les éléments Si et Al, le cation hexylquinuclidinium, et présentant un rapport Si/Al de 11,7 illustre la présente invention.

### EXEMPLE 5 (invention) : Zéolithe de type structural EUO contenant les éléments Si et AI, de rapport Si/Al égal à 27,2 synthétisée avec les précurseurs du bromure d'hexylquinuclidinium comme structurant organique.

Les conditions de synthèse sont définies dans le tableau 7.

On prépare la solution A composée de silicium et des précurseurs de structurant en diluant 1,95 g de quinuclidine (Sigma-Aldrich) et 2,899 g de bromure d'hexyl (Sigma-Aldrich) dans 46,895 g d'eau puis en ajoutant 15,814 g de sol de silice colloïdale (Ludox HS40, Dupont, 40 % SiO₂). On dissout ensuite 0,430 g d'hydroxyde de sodium solide (Prolabo) et 0,430 g d'aluminate de sodium solide (Prolabo, 46 % Al₂O₃, 33 % Na₂O) dans 15,632 g d'eau pour former la solution B. On ajoute la solution B dans la solution A sous agitation puis 22,673 g d'eau. On mélange jusqu'à homogénéisation. On ajoute finalement 0,633 g de germes de zéolithe EUO, post synthèse, contenant le sodium et le cation hexaméthonium, préparée selon les conditions de l'art antérieur (exemple 1). On fait réagir le mélange résultant dans un autoclave de 125 ml sous agitation pendant 10 jours à 180°C sous pression autogène. Après refroidissement, on filtre le produit et on le lave avec 100 ml d'eau déminéralisée puis on le sèche en étuve ventilée à 120°C.

**Tableau 7 : conditions de synthèse**

| Formulation du gel | |
|---|---|
| SiO₂ (mol) | 60 |
| Al₂O₃ (mol) | 1,1 |
| Na₂O (mol) | 5 |
| Quinuclidine (mol) | 10 |
| HexylBr (mol) | 10 |
| H₂O (mol) | 3000 |
| EUO/SiO₂ (g/g) | 0,10 |

| Conditions de cristallisation | |
|---|---|
| Température (°C) | 180 |
| durée (jour) | 10 |

| | |
|---|---|
| HexylBr = bromure d'hexyle | |

Les résultats de diffraction des rayons X, d'analyse chimique sont reportés dans le tableau 8. Cette synthèse conduit à la zéolithe EUO pure, de cristallinité de 90 % par rapport à la référence (solide de l'exemple 1 ayant été utilisé en tant que germes), de rapport Si/Al de 27,2 et contenant le cation hexylquinuclidinium dans sa porosité intracristalline.

**Tableau 8 : caractéristiques du solide obtenu**

| Diffraction des rayons X | |
|---|---|
| Identification de phase | EUO |
| Cristallinité (%) | 90 |

| Analyses chimiques | |
|---|---|
| SiO₂ (% poids) | 71,7 |
| Al₂O₃ (% poids) | 2,24 |
| Na₂O (% poids) | 0,61 |
| N (% poids) | 1,50 |
| PAF (% poids) | 25,4 |
| N/Si (mol/mol) | 0,089 |
| Si/Al (mol/mol) | 27,2 |

Cette zéolithe brute de synthèse de type structural EUO, contenant les éléments Si et Al, le cation hexylquinuclidinium, et présentant un rapport Si/Al de 27,2 illustre la présente invention.

### EXEMPLE 6: préparation de catalyseurs avec les zéolithes de type structural EUO synthétisées dans le système Si-Al, avec HM, DBDMA et l'hexylquinuclidinium, et évaluation catalytique.

Les matières premières utilisées dans cet exemple sont les zéolithes de type structural EUO brutes de synthèse dans le système Si-Al de l'exemple 1 comprenant de l'hexaméthonium (HM) et de rapport atomique Si/Al global égal à 25,0, de l'exemple 2 comprenant du dibenzyldiméthylammonium (DBDMA) et de rapport atomique Si/Al global égal à 26,0 et de l'exemple 5 comprenant de l'hexylquinuclidinium (HexylQ) et de rapport atomique Si/Al global égal à 27,2.

Ces zéolithes de type structural EUO subissent tout d'abord une calcination dite sèche à 550°C sous flux d'air durant 6 heures. Puis les solides obtenus sont soumis à trois échanges ioniques dans une solution de nitrate d'ammonium NH₄NO₃ 10 N, à environ 100°C pendant 4 heures pour chaque échange. Entre chaque échange, les zéolithes sont séchées une nuit dans une étuve à 100°C.

A l'issue de ces traitements, les zéolithes de type structural EUO sous forme NH₄ ont un rapport Si/Al atomique global égal à respectivement 25,2, 26,1 et 27,3 et une teneur pondérale en sodium par rapport au poids de la zéolithe respectivement de 38, 37 et 34 ppm.

Sur les zéolithes précédemment obtenues, 1 % poids de platine est déposé par imprégnation à sec en utilisant comme précurseur une solution aqueuse du composé platine tétramine Pt(NH₃)₄Cl₂, suivie d'une calcination sous air à 450°C et d'une réduction à 500°C sous hydrogène pur.

Les catalyseurs bifonctionnels obtenus sont testés en hydroconversion du n-heptane (hydroisomérisation) dans les conditions suivantes: rapport molaire H₂/n-heptane = 2, pression totale de 1 bar, pph (débit de n-heptane en gramme par gramme de catalyseur et par heure) de 1.

Les résultats obtenus sur les trois catalyseurs sont détaillés au tableau 9 :

**Tableau 9 : évaluation catalytique**

| Nature des éléments de la zéolithe et rapport atomique | Si/Al 25,0 (comp) | Si/Al 26,0 (comp.) | Si/Al 27,2 (invention) |
|---|---|---|---|
| Structurant organique utilisé pour la synthèse de la zéolithe | HM | DBDMA | HexylQ |
| Température nécessaire pour obtenir 80% de conversion du n-heptane (°C) | 230 | 230 | 230 |
| Sélectivité en isomères C7 à 80% de conversion du n-heptane (%) | 63 | 61 | 68 |
| Sélectivité craquage (C1-C6) à 80% de conversion du n-heptane (%) | 37 | 39 | 32 |

Les trois catalyseurs zéolithiques présentent la même activité (même température pour atteindre 80% de conversion du n-heptane). Cependant le catalyseur contenant la zéolithe de type structural EUO. synthétisée avec le structurant organique hexylquinuclidinium (HexylQ), permet d'obtenir un catalyseur plus sélectif (moins de perte par craquage).

### EXEMPLE 7: préparation de catalyseurs avec les zéolithes de type structural EUO synthétisées avec l'hexylquinuclidinium, dans le système Si-Al ou Si-B.

Les matières premières utilisées dans cet exemple sont la zéolithe de type structural EUO brute de synthèse de l'exemple 3 comprenant de l'hexylquinuclidinium (HexylQ) et une charpente composée de silicium et de bore avec un rapport atomique global Si/B de 11,7, et la zéolithe de type structural EUO brute de synthèse de l'exemple 4 comprenant de l'hexylquinuclidinium (HexylQ) et une charpente composée de silicium et d'aluminium avec un rapport atomique global Si/Al de 11,7 également.

Ces zéolithes de type structural EUO subissent tout d'abord une calcination dite sèche à 550°C sous flux d'air durant 6 heures. Puis les solides obtenus sont soumis à trois échanges ioniques dans une solution de nitrate d'ammonium NH₄NO₃ 10 N, à environ 100°C pendant 4 heures pour chaque échange. Entre chaque échange, les zéolithes sont séchées une nuit dans une étuve à 100°C.

A l'issue de ces traitements, les zéolithes de type structural EUO sous forme NH₄ sont caractérisées, pour la zéolithe contenant du silicium et du bore par un rapport Si/B atomique global égal à 12,5 , et pour la zéolithe contenant du silicium et de l'aluminium par un rapport Si/Al atomique global égal à 12,3 et une teneur pondérale en sodium par rapport au poids de la zéolithe égal à 45 ppm.

Sur les zéolithes précédemment obtenues, 1 % poids de platine est déposé par imprégnation à sec en utilisant comme précurseur une solution aqueuse du composé platine tétramine Pt(NH₃)₄Cl₂, suivie d'une calcination sous air à 450°C et d'une réduction à 500°C sous hydrogène pur.

Les catalyseurs bifonctionnels obtenus sont testés en hydroconversion (hydroisomérisation) du n-heptane dans les conditions suivantes: rapport molaire H₂/n-heptane = 2, pression totale de 1 bar, pph (débit de n-heptane en gramme par gramme de catalyseur et par heure) de 1.

Les résultats obtenus sur les deux catalyseurs sont détaillés au tableau 10 :

**Tableau 10 : évaluation catalytique**

| Nature des éléments de la zéolithe et rapport atomique | Si-Al 12,3 (invention) | Si-B 12,5 (comparatif) |
|---|---|---|
| Structurant organique utilisé pour la synthèse de la zéolithe | HexylQ | HexylQ |
| Temp. nécessaire pour obtenir 80% de conversion du n-heptane (°C) | 210 | 300 |
| Sélectivité en isomères C7 à 80% de conversion du n-heptane | 78 | 55 |
| Sélectivité craquage (C1-C6) à 80% de conversion du n-heptane | 22 | 45 |

Le catalyseur contenant la zéolithe de type structural EUO dans le système Si-B est beaucoup moins actif que celui contenant la zéolithe selon l'invention, synthétisée dans le système Si-Al (température nécessaire pour obtenir 80% de conversion du n-heptane plus élevée), et également beaucoup moins sélectif (forte sélectivité en craquage).

## Revendications

1. Utilisation d'une zéolithe de type structural EUO contenant au moins du silicium et au moins de l'aluminium, comme solide acide dans un catalyseur contenant au moins ladite zéolithe sous forme calcinée, au moins un métal du groupe VIII et au moins un liant, ladite zéolithe, sous sa forme brute de synthèse, contenant un cation hexylquinuclidinium dans sa porosité intracristalline et présentant un rapport atomique N/Si supérieur à 0,065, N représentant l'élément azote.

2. Utilisation d'une zéolithe de type structural EUO selon la revendication 1 telle que ladite zéolithe, sous sa forme brute de synthèse, présente un rapport Si/Al compris entre 5 et 50.

3. Utilisation d'une zéolithe de type structural EUO selon la revendication 2 telle que ladite zéolithe, sous sa forme brute de synthèse, présente un rapport Si/Al compris entre 6 et 40.

4. Utilisation d'une zéolithe de type structural EUO selon la revendication 3 telle que ladite zéolithe, sous sa forme brute de synthèse, présente un rapport Si/Al compris entre 7 et 30.

5. Utilisation d'une zéolithe de type structural EUO selon l'une des revendications 1 à 4 telle que ledit catalyseur contient au moins un élément choisi dans le groupe formé par les éléments des groupes IB, IIB, IIIA, IVA, VIB et VIIB.

6. Utilisation d'une zéolithe de type structural EUO selon l'une des revendications 1 à 5 telle que ledit catalyseur contient du soufre.

7. Utilisation d'une zéolithe de type structural EUO selon l'une des revendications 1 à 6 telle que ledit métal du groupe VIII est choisi dans le groupe constitué par le palladium et le platine.

8. Utilisation d'une zéolithe de type structural EUO selon l'une des revendications 1 à 7 telle que ledit liant est l'alumine.

9. Utilisation d'une zéolithe de type structural EUO selon l'une des revendications 1 à 8 comme solide acide dans une réaction d'hydroisomérisation du n-heptane après calcination de ladite zéolithe.

## Claims

1. Use of a EUO-structural-type zeolite that comprises at least silicon and and at least aluminum as an acidic solid in a catalyst comprising at least said zeolite in its calcined form, at least a GVIII metal and at least a binder, said zeolite containing in its crude synthesis form at least one alkyl quinuclidinium cation in its intracrystalline pores and in that it has an N/Si atomic ratio that is higher than 0.465, whereby N represents the nitrogen element

2. Use of a EUO-structural-type zeolite according to claim 1, wherein said zeolite in its it crude synthesis form has an Si/Al ratio of between 5 and 50.

3. Use of a EUO-structural-type zeolite according to claim 2, wherein said zeolite in its it crude synthesis form has an Si/Al ratio of between 6 and 40.

4. Use of a EUO-structural-type zeolite according to claim 3, wherein said zeolite in its it crude synthesis form has an Si/Al ratio of between 7 and 30.

5. Use of a EUO-structural-type zeolite according to one of claims 1 to 4, wherein said catalyst contains at meast one element selectd from the group that is formed by the elements of groups IB, IIB, IIIA, IVA, VIB and VIIB.

6. Use of a EUO-structural-type zeolite according to one of claims 1 to 5, wherein said catalyst contains sufur.

7. Use of a EUO-structural-type zeolite according to one of claims 1 to 6, wherein said group VIII metal is chosen among palladium and platinum.

8. Use of a EUO-structural-type zeolite according to one of claims 1 to 7, wherein said binder is alumina.

9. Use of a EUO-structural-type zeolite according to one of claims 1 to 8, as an acidic solid in a reaction for hydroisomerization of n-heptane after calcination, of said zeolite.

## Patentansprüche

1. Verwendung eines Zeoliths vom Strukturtyp EUO, der mindestens Silicium und mindestens Aluminium enthält, als saurer Feststoff in einem Katalysator, welcher mindestens den Zeolith in gebrannter Form, mindestens ein Metall der Gruppe VIII und mindestens ein Bindemittel enthält, wobei der Zeolith in seiner unbehandelten syntheseform ein Hexylquinuclidinium-Kation in seinem intrakristallinen Porenraum enthält und ein N/Si-Atomverhältnis von mehr als 0,065 aufweist, wobei N für das Element Stickstoff steht.

2. Verwendung eines Zeoliths vom Strukturtyp EUO nach Anspruch 1, derart, dass der Zeolith in seiner unbehandelten Syntheseform ein Si/Al-Verhältnis im Bereich von 5 bis 50 aufweist.

3. Verwendung eines Zeoliths vom Strukturtyp EUO nach Anspruch 2, derart, dass der Zeolith in seiner unbehandelten Syntheseform ein Si/Al-Verhältnis im Bereich von 6 bis 40 aufweist.

4. Verwendung eines Zeoliths vom Strukturtyp EUO nach Anspruch 3, derart, dass der Zeolith in seiner unbehandelten Syntheseform ein Si/Al-Verhältnis im Bereich von 7 bis 30 aufweist.

5. Verwendung eines Zeoliths vom Strukturtyp EUO nach einem der Ansprüche 1 bis 4, derart, dass der Katalysator mindestens ein Element enthält, das aus der Gruppe ausgewählt ist, welche von den Elementen der Gruppen IB, IIB, IIIA, IVA, VIB und VIIB gebildet wird.

6. Verwendung eines Zeoliths vom Strukturtyp EUO nach einem der Ansprüche 1 bis 5, derart, dass der Katalysator Schwefel enthält.

7. Verwendung eines Zeoliths vom Strukturtyp EUO nach einem der Ansprüche 1 bis 6, derart, dass das Metall der Gruppe VIII aus der Gruppe ausgewählt ist, welche aus Palladium und Platin besteht.

8. Verwendung eines Zeoliths vom Strukturtyp EUO nach einem der Ansprüche 1 bis 7, derart, dass es sich bei dem Bindemittel um Aluminiumoxid handelt.

9. Verwendung eines Zeoliths vom Strukturtyp EUO nach einem der Ansprüche 1 bis 8 als saurer Feststoff in einer Reaktion zur Hydroisomerisierung von n-Heptan, nach dem Brennen des Zeoliths.
